# EUROPEAN PATENT APPLICATION

(11) **EP 4 403 913 A1**
(43) Date of publication of application: **24.07.2024**
(21) Application number: 22869567.2
(22) Date of filing: 26.01.2022
(51) Int. Cl.: G01N 31/00, G01N 31/12

(54) **TOTAL ORGANIC CARBON ANALYZER**

(30) Priority: 17.09.2021 JP 2021152061
(71) Applicant: Shimadzu Corporation, Nakagyo-ku, Kyoto-shi, Kyoto 604-8511 (JP)
(72) Inventor: TANAKA, Minako, Kyoto-shi, Kyoto 604-8511 (JP)
(74) Representative: Müller-Boré & Partner Patentanwälte PartG mbB
(86) International application number: PCT/JP2022/002886
(87) International publication number: WO 2023/042414

(57) **Abstract**

A total organic carbon analyzer (1) including a combustion unit (21) configured to combust a liquid sample in the presence of a heated oxidation catalyst (212), a liquid supply unit (10) configured to switch between a measurement target liquid sample and pure water to supply to the combustion unit (21), and a carbon dioxide amount measurement unit (24) configured to measure an amount of carbon dioxide generated in the combustion unit (21), the total organic carbon analyzer (1) further comprising:
a start operation reception unit (31) configured to receive a predetermined start operation by a user;
a device activation processing unit (32) configured to execute activation processing of activating the combustion unit (21) and the carbon dioxide amount measurement unit (24) when the start operation reception unit (31) receives a start operation; and
a blank measurement execution unit (33) configured to, after a predetermined time elapses from the start of the startup process or after it is detected that the combustion unit (21) reaches a predetermined temperature and the carbon dioxide amount measurement unit (24) is in a stable state, start a pure water measurement operation of supplying the pure water from the liquid supply unit (10) to the combustion unit (21) and measuring the amount of carbon dioxide in the carbon dioxide amount measurement unit (24), and perform control of repeatedly executing the pure water measurement operation until measured values of the amount of carbon dioxide in a latest predetermined multiple rounds fall within a predetermined range.

## Description

### TECHNICAL FIELD

The present invention relates to a total organic carbon analyzer.

### BACKGROUND ART

Total Organic Carbon (TOC) is an index representing the total amount of organic substances present in a liquid sample such as wastewater or environmental water by the amount of carbon contained in the organic substances, and the total organic carbon analyzer is a device configured to measure TOC. The total organic carbon analyzer, for example as described in Patent Literature 1, combusts a liquid sample by supplying the liquid sample together with a carrier gas (oxygen or high purity air) to a combustion tube filled with an oxidation catalyst, and measures the amount of CO₂ (carbon dioxide) derived from organic substances in the liquid sample contained in the gas generated by the combustion using a CO₂ measurement device such as a non-dispersive infrared absorption analyzer, to obtain TOC.

In order to stably operate the total organic carbon analyzer, it is necessary to heat the combustion tube to a predetermined temperature (for example, 680 °C), and to activate the CO₂ measurement device in advance. It usually takes 30 minutes or more for the total organic carbon analyzer to be in a stable state. Therefore, a normal total organic carbon analyzer is provided with an automatic activation function, and when a user first performs a predetermined activation operation such as pressing of a button, heating of the combustion tube is started and the CO₂ measurement device is activated. After a predetermined time elapses or after it is detected that the combustion tube reaches a predetermined temperature and the CO₂ measurement device is in a stable state, a state is established in which measurement on the liquid sample can be started.

In the CO₂ measurement device of the total organic carbon analyzer, usually, the detection signal does not become 0 even if CO₂ is not present due to an influence of noise or the like. The detection value of the CO₂ measurement device when CO₂ is not present as described above is referred to as system blank value. Therefore, in order to obtain the total amount of organic substances in the liquid sample using the total organic carbon analyzer, it is necessary to subtract the system blank value from the detection value of the CO₂ measurement device at the time of measuring the liquid sample. Therefore, in the total organic carbon analyzer, after the state is established in which the measurement can be started as described above, the system blank value is usually measured (blank measurement) before the measurement of the liquid sample is started. In the blank measurement, pure water for blank measurement having a low organic impurity concentration is prepared, and operation of supplying the pure water for blank measurement to the combustion tube instead of the liquid sample, and heating the pure water for blank measurement to generate gas, and measuring the generated gas with the CO₂ measurement device is performed repeatedly for a plurality of times. In an initial stage of the plurality of times of measurements, molecules of CO₂ adhering to the combustion tube, the oxidation catalyst, and the like are also detected in addition to CO₂ still remaining in the pure water for blank measurement (therefore, blank measurements in the initial stage among the blank measurements serve also as washing the combustion tube, the oxidation catalyst, and the like), but the measured value decreases while the measurement is repeatedly performed, and becomes stable at a substantially constant value. The measured value in the pure water for blank measurement, which is thus stable, is set as the system blank value, and a value obtained by subtracting the system blank value from a measured value of a liquid sample executed after that is regarded as the amount of CO₂ derived from the organic substance in the liquid sample.

In a device described in Patent Literature 1, after the total organic carbon analyzer is activated to be in a stable state, when a user performs a start operation of blank measurement, measurement of pure water for blank measurement is automatically repeated until the measured value becomes stable at a predetermined value or less.

### CITATION LIST

### PATENT LITERATURE

Patent Literature 1: JP 2001-318089 A

### SUMMARY OF INVENTION

### TECHNICAL PROBLEM

Usually, blank measurement takes about several tens of minutes. In the device described in Patent Literature 1, the user has to wait for several tens of minutes after the user performs the start operation of the blank measurement until the blank measurement is completed, and then the user has to perform the measurement operation of the liquid sample to be measured again, which takes time and labor.

A problem to be solved by the present invention is to provide a total organic carbon analyzer allowing a user to reduce time and effort required for measuring a sample.

### SOLUTION TO PROBLEM

The present invention made to solve the above problems is a total organic carbon analyzer including a combustion unit configured to combust a liquid sample in the presence of a heated oxidation catalyst, a liquid supply unit configured to switch between a measurement target liquid sample and pure water to supply to the combustion unit, and a carbon dioxide amount measurement unit configured to measure an amount of carbon dioxide generated in the combustion unit, the total organic carbon analyzer further including:
a start operation reception unit configured to receive a predetermined start operation by a user;
a device activation processing unit configured to execute activation processing of activating the combustion unit and the carbon dioxide amount measurement unit when the start operation reception unit receives a start operation; and
a blank measurement execution unit configured to, after a predetermined time elapses from the start of the startup process or after it is detected that the combustion unit reaches a predetermined temperature and the carbon dioxide amount measurement unit is in a stable state, start a pure water measurement operation of supplying the pure water from the liquid supply unit to the combustion unit and measuring the amount of carbon dioxide in the carbon dioxide amount measurement unit, and perform control of repeatedly executing the pure water measurement operation until measured values of the amount of carbon dioxide in a latest predetermined multiple rounds fall within a predetermined range.

In the present invention, the "predetermined time" is a time normally required for the combustion unit to reach a predetermined temperature and the carbon dioxide amount measurement unit to be in a stable state, or a time longer than that. The "predetermined temperature" is a temperature when pure water or a liquid sample is measured. The "latest predetermined multiple rounds" and the "predetermined range" of the measured value of the amount of carbon dioxide in the pure water measurement operation may be determined as appropriate on the basis of the number of measurement times of pure water when the system blank value is obtained in a normal total organic carbon analyzer and a range of variation in which the measured value is determined to be stable. Alternatively, these values may be set by being input by the user.

### ADVANTAGEOUS EFFECTS OF INVENTION

With the total organic carbon analyzer according to the present invention, when the user executes only the operation of starting the total organic carbon analyzer, the blank measurement is automatically performed following the activation processing of a total organic carbon analyzer device body. This allows the user to reduce the time and effort required to perform the measurement of the sample (to the measurement start of the sample).

### BRIEF DESCRIPTION OF DRAWINGS

[Fig. 1] Fig. 1 is a schematic configuration diagram illustrating an embodiment of a total organic carbon analyzer according to the present invention.
[Fig. 2] Fig. 2 is a flowchart illustrating an operation of the total organic carbon analyzer of the present embodiment.
[Fig. 3] Fig. 3 is a schematic configuration diagram illustrating an operation of a first modification of the present embodiment.
[Fig. 4] Fig. 4 is a flowchart illustrating an operation of the first modification.
[Fig. 5] Fig. 5 is a schematic configuration diagram illustrating a total organic carbon analyzer of a second modification of the present embodiment.
[Fig. 6] Fig. 6 is a flowchart illustrating an operation of the total organic carbon analyzer of the second modification.
[Fig. 7] Fig. 7 is a diagram illustrating an example of a screen displayed on a display unit at the time of activation in the total organic carbon analyzer of the second modification.

### DESCRIPTION OF EMBODIMENTS

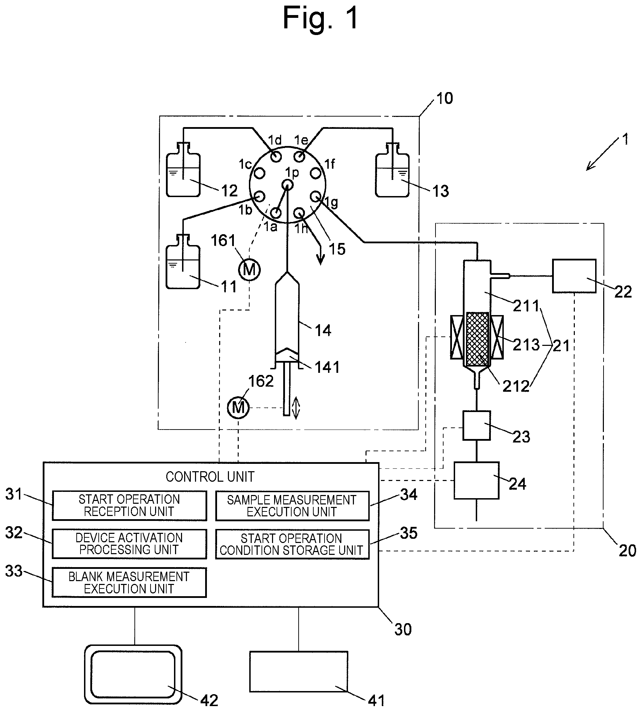

Embodiments of a total organic carbon analyzer according to the present invention will be described with reference to Figs. 1 to 7. As illustrated in Fig. 1, a total organic carbon analyzer 1 of the present embodiment includes a liquid supply unit 10, a total organic carbon measurement unit 20, and a control unit 30.

The liquid supply unit 10 includes a measurement target liquid sample reservoir 11 configured to store a liquid sample to be measured (measurement target liquid sample), a pure water reservoir 12 configured to store pure water, a dilute hydrochloric acid reservoir 13 configured to store dilute hydrochloric acid as an inorganic acid solution, a syringe 14, and an eight-way valve 15. Here, pure water for blank measurement having a low organic impurity concentration is used as pure water stored in the pure water reservoir 12.

Each of the measurement target liquid sample reservoir 11, the pure water reservoir 12, and the dilute hydrochloric acid reservoir 13 is connected to one of the eight ports 1a to 1h of the eight-way valve 15. In the example of Fig. 1, the measurement target liquid sample reservoir 11 is connected to the port 1b, the pure water reservoir 12 is connected to the port 1d, and the dilute hydrochloric acid reservoir 13 is connected to the port 1e. In addition, an inlet of a combustion tube 21 of a total organic carbon measurement unit 20 described later is connected to one of the eight ports 1a to 1h (a port 1g in the example of Fig. 1). The syringe 14 is connected to a common port 1p of the eight-way valve 15. One of the eight ports 1a to 1h (the port 1h in the example of Fig. 1) functions as a drain configured to discharge waste water generated by washing the syringe 14 with pure water supplied from the pure water reservoir 12 to the syringe 14. A plurality of the measurement target liquid sample reservoirs 11 may be provided and correspondingly connected to different ports among the ports 1a to 1h.

The eight-way valve 15 is provided with a first motor 161. When the eight-way valve 15 is rotated by the operation of the first motor 161, the ports connected to the common port 1p among the ports 1a to 1h are switched. In addition, the syringe 14 is provided with a conversion mechanism (not illustrated) configured to convert the rotational motion of a second motor 162 and the second motor 162 into a reciprocating motion, and allow a piston 141 of the syringe 14 to reciprocate when the second motor 162 rotates in a forward rotational direction and a reverse rotational direction.

The total organic carbon measurement unit 20 includes a combustion unit 21, a carrier gas introduction unit 22, a dehumidification/gas treatment unit 23, and a carbon dioxide amount measurement unit 24. The combustion unit 21 is made of a combustion tube 211, an oxidation catalyst 212 disposed inside the combustion tube 211, and an electric furnace 213 provided around the combustion tube 211. The electric furnace 213 has an ability to heat the oxidation catalyst 212 in the combustion tube 211 to 680 °C. The carrier gas introduction unit 22 is connected to the combustion tube 211, and introduces a carrier gas made of high-purity air or oxygen into the combustion tube 21. The dehumidification/gas treatment unit 23 is a device connected to an outlet of the combustion tube 211, and configured to remove water vapor contained in the gas generated in the combustion tube 211. The carbon dioxide amount measurement unit 24 is connected to an outlet of treated gas in the dehumidification/gas treatment unit 23, and in the present embodiment, a non-dispersive infrared absorption analyzer is used.

The control unit 30 controls the operation of the total organic carbon analyzer 1, and is embodied by combining hardware such as a CPU and a memory with a control program (software). The control unit 30 includes, as functional blocks, a start operation reception unit 31, a device activation processing unit 32, a blank measurement execution unit 33, a sample measurement execution unit 34, and a start operation condition storage unit 35. The functions of these units will be described later together with the overall operation of the total organic carbon analyzer 1.

The total organic carbon analyzer 1 further includes an input unit 41 and a display unit 42. The input unit 41 is a device to which a user inputs predetermined pieces of information, and a keyboard, a touch panel, and the like is applied. The display unit 42 is a monitor configured to display a blank measurement result, a measurement result of the measurement target liquid sample, and the like.

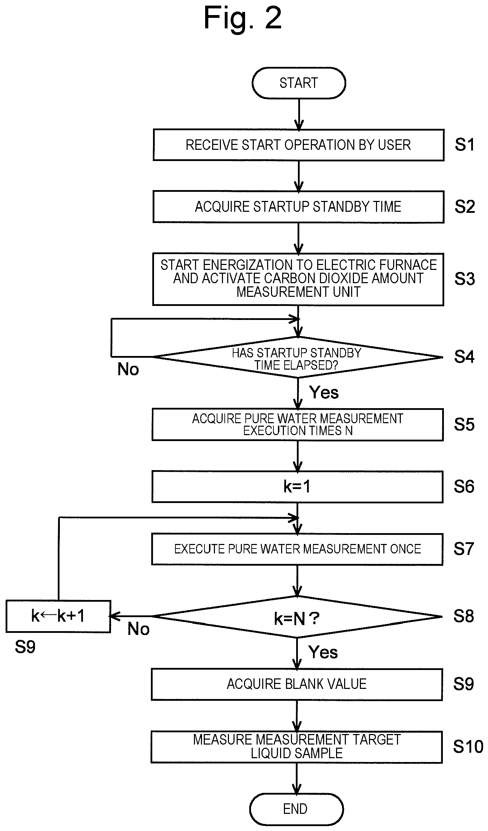

Hereinafter, the operation of the total organic carbon analyzer 1 of the present embodiment will be described with reference to Fig. 2.

In a state where the main power of the total organic carbon analyzer 1 is on, when the user executes an operation for starting the activation processing using the input unit 41, the start operation reception unit 31 receives a signal indicating that the activation processing start operation has been executed (step 1), and transmits a signal that causes each unit in the control unit 30 to execute the activation processing. Examples of the processing start operation executed by the user include pressing a predetermined key among keys in the keyboard as the input unit 41 and keys displayed on the touch panel. Alternatively, apart from the keyboard or the like, the total organic carbon analyzer 1 may be provided with an activation switch.

Upon receiving a signal from the start operation reception unit 31, the device activation processing unit 32 acquires a startup standby time as a start operation condition from the start operation condition storage unit 35 (step 2). The startup standby time is a waiting time after the device activation processing unit 32 starts an activation operation of the device until the blank measurement execution unit 33 starts blank measurement, and is a time set as a necessary and sufficient time until the combustion tube 211 and the oxidation catalyst 212 of the combustion unit 21 reach their respective predetermined temperatures and the carbon dioxide amount measurement unit 24 becomes stable. The device activation processing unit 32 starts energization to the electric furnace 213 and executes an operation of activating the carbon dioxide amount measurement unit 24 (step 3) to start counting of time.

When the time is counted until the startup standby time elapses (Yes in step 4), it is estimated that the combustion tube 211 and the oxidation catalyst 212 of the combustion unit 21 each reach the predetermined temperature and the carbon dioxide amount measurement unit 24 is in the stable state, so that the device activation processing is ended and the processing proceeds to blank measurement in step 5 and subsequent steps. Here, since the processing proceeds to execution of the blank measurement automatically after completion of the activation operation, the user does not have to perform an operation for executing the blank measurement again. After the startup standby time has elapsed, the device activation processing unit 32 may acquire a measured value of a temperature of each unit in the combustion unit 21 and measured data of the carbon dioxide amount measurement unit 24, and may confirm whether the measurement results satisfy the condition for ending the device activation processing.

In step 5, the blank measurement execution unit 33 acquires the number of times of executing blank measurement as the start operation condition from the start operation condition storage unit 35. This number of blank measurement execution times N (N is a natural number) is a value set as a necessary and sufficient number of times such that the blank measurement is repeatedly executed and the amount of carbon dioxide measured by the carbon dioxide amount measurement unit 24 in the latest predetermined multiple rounds falls within a predetermined range. The blank measurement execution unit 33 sets k = 1 as an initial condition (step 6) and executes blank measurement once (step 7).

In the blank measurement, first, introduction of the carrier gas from the carrier gas introduction unit 22 into the combustion tube 211 is started. The carrier gas is continuously supplied to the combustion tube 211 during the blank measurement.

Next, when the eight-way valve 15 rotates by the operation of the first motor 161, the port 1d is connected to the common port 1p. In this state, when the piston 141 is retracted by a predetermined distance by the operation of the second motor 162, pure water in the pure water reservoir 12 is sucked into the syringe 14 by a predetermined amount through the port 1d and the common port 1p. Next, when the eight-way valve 15 rotates by the operation of the first motor 161, the port 1g is connected to the common port 1p. In this state, when the piston 141 advances by the operation of the second motor 162, the pure water in the syringe 14 is introduced to the combustion tube 211 through the common port 1p and the port 1g.

In the combustion tube 211, the pure water is vaporized by being heated, and water vapor is generated. This water vapor contains CO₂ obtained by combusting organic substances slightly contained in the pure water. In addition, molecules of CO₂ adhering to the inside of the combustion tube 211 and the oxidation catalyst 212 are desorbed and taken into the water vapor. The gas containing CO₂ in the water vapor thus generated is introduced into the dehumidification/gas treatment unit 23 and most of the water vapor is removed, and CO₂ (and water vapor that cannot be removed by the dehumidifying/gas treatment unit 23 and remains partially) is introduced into the carbon dioxide amount measurement unit 24. The carbon dioxide amount measurement unit 24 measures the amount of carbon dioxide in the introduced gas.

The above operation has caused pure water measurement to be completed once. Normally, when the pure water measurement is performed only once, CO₂ molecules adhering to the combustion tube 211, the oxidation catalyst 212, and the like are also detected by the carbon dioxide amount measurement unit 24, and thus the measured value of the carbon dioxide amount measurement unit 24 becomes a value higher than that in the predetermined range.

When a value of k is less than N (No in step 8), the blank measurement execution unit 33 adds 1 to the value of k (step 9), returns to step 7, and executes the pure water measurement once again. In this way, when the pure water measurement is repeatedly executed N times and k = N is established (Yes in step 8), it is estimated that the measured value of the carbon dioxide amount measurement unit 24 falls within the predetermined range, and thus the most-recent plurality of times of measured values are acquired as blank values (step 10), and the blank measurement is completed.

Then, the processing proceeds to step 11, and the sample measurement execution unit 34 executes TOC measurement on the measurement target liquid sample. The TOC measurement of the measurement target liquid sample is started in a state where the introduction of the carrier gas into the combustion tube 211, the heating of the combustion tube 211 and the oxidation catalyst 212, and the operation of the carbon dioxide amount measurement unit 24 are continued from the time of the blank measurement.

In the TOC measurement of the measurement target liquid sample, first, when the eight-way valve 15 rotates by the operation of the first motor 161, the port 1b is connected to the common port 1p. In this state, when the piston 141 is retracted by a predetermined distance by the operation of the second motor 162, the measurement target liquid sample in the measurement target liquid sample reservoir 11 is sucked into the syringe 14 by a predetermined amount through the port 1b and the common port 1p. Next, when the eight-way valve 15 rotates by the operation of the first motor 161, the port 1g is connected to the common port 1p. In this state, when the piston 141 advances by the operation of the second motor 162, the measurement target liquid sample in the syringe 14 is introduced to the combustion tube 211 through the common port 1p and the port 1g. In the combustion tube 211, when the organic substance in the measurement target liquid sample combusts to generate CO₂, and the amount of CO₂ is measured by the carbon dioxide amount measurement unit 24, a value of TOC in the measurement target liquid sample is obtained.

When there is a plurality of measurement target liquid samples, the above-described TOC measurement of the measurement target liquid sample is repeatedly executed. Then, upon completion of the measurement for all the measurement target liquid samples, a series of operations of the total organic carbon analyzer 1 ends.

According to the total organic carbon analyzer 1 of the present embodiment, when the user executes only the operation of starting the present total organic carbon analyzer 1 (step 1), the blank measurement (steps 5 to 9) is automatically executed following the activation processing (steps 2 to 4) of the total organic carbon analyzer device body. This allows the user to reduce time and effort required for the operation to the measurement start (step 10) of the measurement target liquid sample.

In addition, pieces of measurement data of both of the blank measurement and the measurement target liquid sample are obtained by the carbon dioxide amount measurement unit 24, but since the blank measurement is performed separately from the measurement of the measurement target liquid sample, there is no possibility that the measurement data acquired by the blank measurement and the measurement data acquired by the measurement target liquid sample are confused.

In the total organic carbon analyzer 1 of the present embodiment, as one of the start operation conditions, dilute hydrochloric acid may be introduced into the combustion tube 211 in place of pure water to perform the measurement in the measurement performed before the last plurality of times used for obtaining the blank value among the plurality of times of measurement in the blank measurement. As a result, when impurities adhere to the combustion tube 211, the oxidation catalyst 212, or an inner wall surface of a tube in front of them through which the measurement target liquid sample flows, dissolution and removal of the impurities can be promoted by dilute hydrochloric acid, and influence of the impurities at the time of measurement of the measurement target liquid sample can be suppressed. In addition, although the function of the oxidation catalyst 212 may deteriorate after alkaline measurement target liquid sample is measured, the function of the oxidation catalyst 212 can be recovered by passing dilute hydrochloric acid. When the measurement of dilute hydrochloric acid is performed, dilute hydrochloric acid may be supplied from the dilute hydrochloric acid reservoir 13 to the syringe 14 by connecting the port 1e to the common port 1p instead of connecting the port 1d to the common port 1p which is performed at the time of the measurement of pure water.

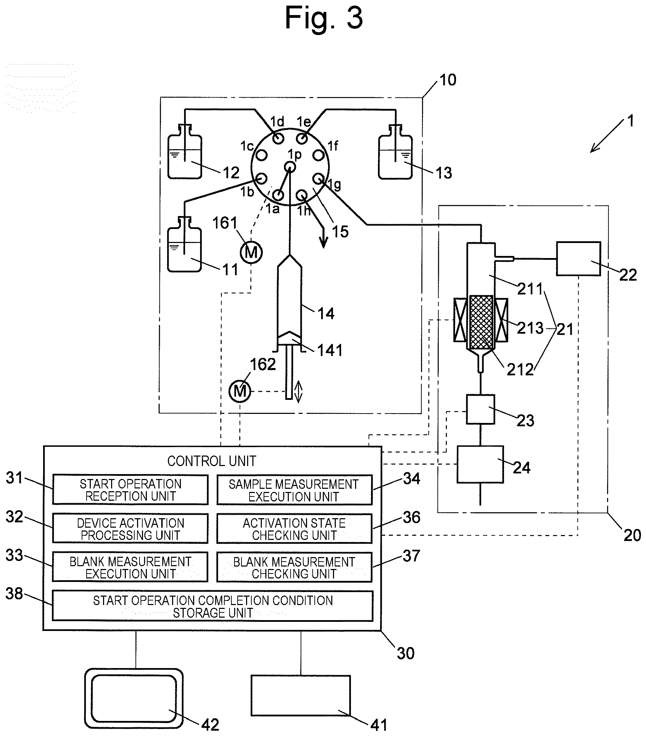

Fig. 3 is a configuration of a first modification in the total organic carbon analyzer of the present embodiment. In the first modification, the control unit 30 functionally includes an activation state checking unit 36, a blank measurement checking unit 37, and a start operation completion condition storage unit 38 instead of the start operation condition storage unit 35 in the above-described embodiment. The functions of these additional units will be described later together with the overall operation of the total organic carbon analyzer 1 of the first modification. Other configurations of the first modification are the same as those of the total organic carbon analyzer 1 of the above-described embodiment.

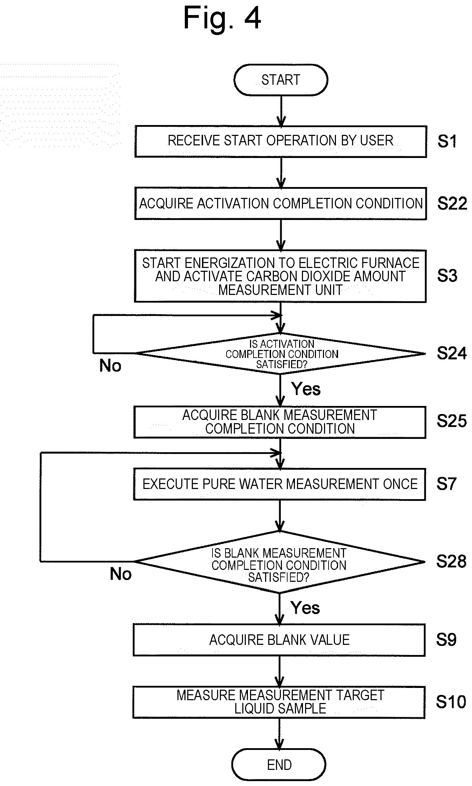

The operation of the total organic carbon analyzer of the first modification is illustrated in Fig. 4. Similarly to the above-described embodiment, after the start operation reception unit 31 receives the signal indicating that the start processing start operation has been executed (step 1), the device activation processing unit 32 acquires, from the start operation completion condition storage unit 38, an activation completion condition for determining that the activation operation of the total organic carbon analyzer has been completed. Examples of the activation completion condition include temperatures of the combustion tube 211 and the oxidation catalyst 212 of the combustion unit 21, and measured values of the carbon dioxide amount measurement unit 24. After the device activation processing unit 32 executes the activation operation (step 3) by the same method as the above-described embodiment, the activation state checking unit 36 acquires the temperatures of the combustion tube 211 and the oxidation catalyst 212 and the measured values of the carbon dioxide amount measurement unit 24 as needed, and determines whether or not the measured values satisfy the activation completion condition acquired from the start operation completion condition storage unit 38 (step 24).

When the activation state checking unit 36 determines that the activation completion condition is satisfied (Yes in step 24), the blank measurement execution unit 33 acquires a blank measurement completion condition for determining that the blank measurement is completed from the start operation completion condition storage unit 38 (step 25). Examples of the blank measurement completion condition include a predetermined value as a measured value of the carbon dioxide amount measurement unit 24 and the number of times of pure water measurement in which the predetermined value is continuously measured. The blank measurement execution unit 33 executes pure water measurement once by the same method as in the above-described embodiment (step 7). After executing the pure water measurement once, the blank measurement checking unit 37 acquires the measured value of the carbon dioxide amount measurement unit 24 and determines whether or not the blank measurement completion condition is satisfied (step 28). When the blank measurement completion condition is not satisfied (No in step 28), the processing returns to step 7 and the pure water measurement is further performed once. When the blank measurement completion condition is satisfied (Yes in step 28), a blank value is acquired (step 9), and the sample measurement execution unit 34 executes measurement of the measurement target liquid sample (step 10). Hereinafter, upon completion of the measurement operation of the measurement target liquid sample similar to that of the above-described embodiment, a series of operations of the total organic carbon analyzer of the first modification ends.

With the total organic carbon analyzer according to the first modification, when the activation state checking unit 36 and the blank measurement checking unit 37 respectively confirm that the completion conditions stored in the start operation completion condition storage unit 38 are satisfied for the activation processing and the blank measurement, the activation processing and the blank measurement are completed, so that the completion conditions can be more reliably satisfied.

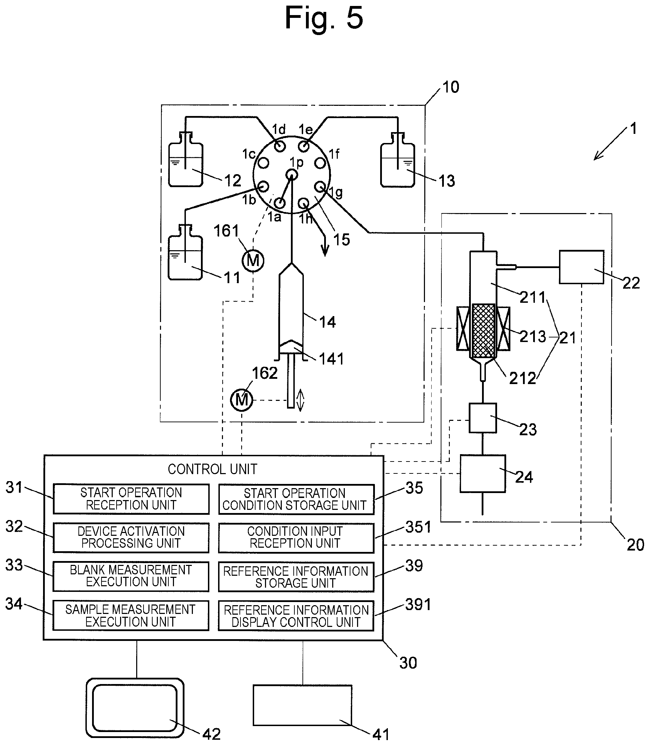

Fig. 5 illustrates a second modification of the total organic carbon analyzer of the present embodiment. In the second modification, the control unit 30 functionally includes a condition input reception unit 351, a reference information storage unit 39, and a reference information display control unit 391 in addition to the units in the above-described embodiment. The functions of these additional units will be described later together with the overall operation of the total organic carbon analyzer of the second modification. Other configurations of the second modification are the same as those of the total organic carbon analyzer 1 of the above-described embodiment.

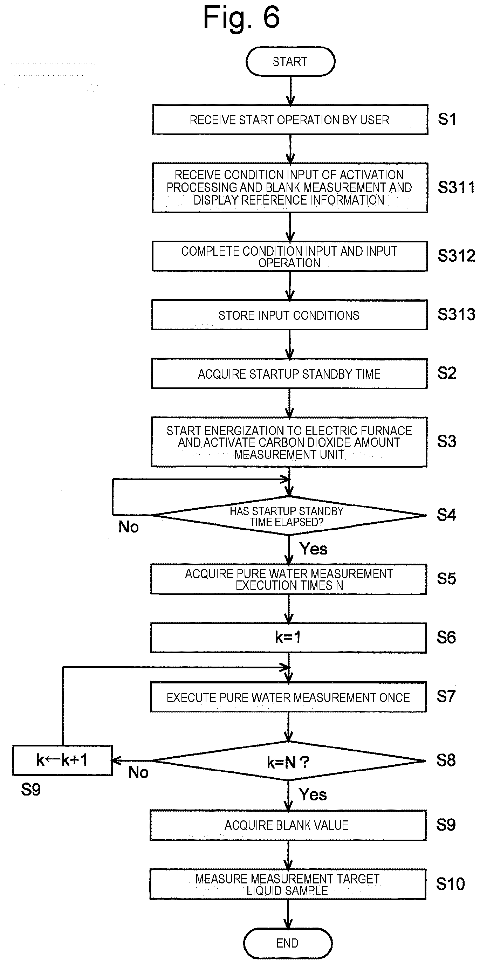

The operation of the total organic carbon analyzer of the second modification is illustrated in Fig. 6. Similarly to the above-described embodiment, after the start operation reception unit 31 receives a signal indicating that the activation processing start operation has been executed (step 1), the following two operations are executed in step 311. First, the condition input reception unit 351 executes processing for allowing the user to input a condition of the activation processing and a condition of the blank measurement. Specifically, the condition input reception unit 351 causes the display unit 42 to display an input field for each item of the condition requiring the user to input, and when the user operates the input unit 41 to input the condition, the condition input reception unit 351 receives the input information. Second, the reference information display control unit 391 executes processing of causing the display unit 42 to display information that is stored in the reference information storage unit 39 and serves as a reference when the user defines the conditions of the activation processing and the conditions of the blank measurement.

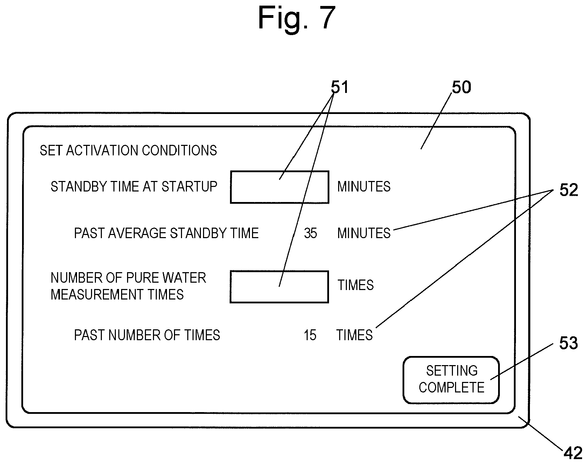

Fig. 7 illustrates an example of a screen displayed in the display unit 42. A screen 50 is provided with an input field 51 in which the user inputs a waiting time (startup standby time) after the device activation processing unit 32 starts the activation operation of the device until the blank measurement execution unit 33 starts the blank measurement, and the number of times of pure water measurement operation executed by the blank measurement execution unit 33. Instead of the startup standby time, a target heating temperatures of the combustion tube 211 and the oxidation catalyst 212 of the combustion unit 21 and a measured value by the carbon dioxide amount measurement unit 24 configured to be able to determine that the carbon dioxide amount measurement unit 24 is in a stable state may be set.

In addition, in the screen 50, as the reference information 52, an average startup time (average value of time required until the combustion unit reaches a predetermined temperature and the carbon dioxide amount measurement unit becomes stable in the past measurement) and an average number of pure water measurement times (average value of the number of times of pure water measurement required until the measured value of the amount of carbon dioxide falls within a predetermined range. Instead of this value, an average value of the number of times of pure water measurement actually performed may be displayed), and a type of a measurement target liquid sample most recently measured are displayed. In addition, a condition setting completion button 53 is displayed on the screen 50.

The user determines the completion condition of the activation operation and the completion condition of the blank measurement while referring to the reference information 52 displayed on the screen 50, and inputs these conditions to the input field 51. After completion of the input, when the user performs an operation of pressing the condition setting completion button 53 (step 312), the condition input reception unit 351 detects the operation and stores the input completion condition of the activation operation and the input completion condition of the blank measurement in the start operation condition storage unit 35 (step 313).

Next, the device activation processing unit 32 acquires, from the start operation condition storage unit 35, the completion condition (the startup standby time in the example of Figs. 6 and 7) of the activation operation input by the user (step 2), and executes the activation operation by the same method as in the above-described embodiment (steps 3 and 4). Subsequently, the blank measurement execution unit 33 acquires the blank measurement completion condition (the number of times of pure water measurement N in the example of Figs. 6 and 7) input by the user from the start operation condition storage unit 35 (step 5), and executes the blank measurement by the same method as in the above-described embodiment (steps 6 to 9). Then, measurement is executed on the measurement target liquid sample by the same method as that in the above-described embodiment (step 10), and a series of operations of the total organic carbon analyzer of the second modification ends.

According to the total organic carbon analyzer of the second modification, since the user inputs the completion condition of the activation processing and the completion condition of the blank measurement by the operation of the condition input reception unit 351, these conditions can be set by the determination of the user. At that time, since pieces of the reference information for the user to set the condition of the activation processing and the condition of the blank measurement stored in the reference information storage unit 39 is displayed on the display unit 42, the user can set the completion conditions of the activation processing and the blank measurement with reference to those pieces of the reference information.

The present invention is not limited to the above-described embodiment and modifications, and can be modified as appropriate within the scope of the gist of the present invention. In addition, components included in each of the above-described embodiment and the two modifications may be combined and used as appropriate.

### [Modes]

It is understood by those skilled in the art that the plurality of exemplary embodiments described above are specific examples of the following modes.

### (Clause 1)

A total organic carbon analyzer according to Clause 1 is a total organic carbon analyzer including a combustion unit configured to combust a liquid sample in the presence of a heated oxidation catalyst, a liquid supply unit configured to switch between a measurement target liquid sample and pure water to supply to the combustion unit, and a carbon dioxide amount measurement unit configured to measure an amount of carbon dioxide generated in the combustion unit, the total organic carbon analyzer further including:
a start operation reception unit configured to receive a predetermined start operation by a user;
a device activation processing unit configured to execute activation processing of activating the combustion unit and the carbon dioxide amount measurement unit when the start operation reception unit receives a start operation; and
a blank measurement execution unit configured to, after a predetermined time elapses from the start of the startup process or after it is detected that the combustion unit reaches a predetermined temperature and the carbon dioxide amount measurement unit is in a stable state, start a pure water measurement operation of supplying the pure water from the liquid supply unit to the combustion unit and measuring the amount of carbon dioxide in the carbon dioxide amount measurement unit, and perform control of repeatedly executing the pure water measurement operation until measured values of the amount of carbon dioxide in a latest predetermined multiple rounds fall within a predetermined range.

With the total organic carbon analyzer according to Clause 1, when the user executes only the operation of starting the total organic carbon analyzer, the blank measurement is automatically performed following the activation processing of a total organic carbon analyzer device body. This allows the user to reduce the time and effort required to perform the measurement of the sample (to the measurement start of the sample).

### (Clause 2)

A total organic carbon analyzer according to Clause 2 further includes, in the total organic carbon analyzer according to item 1, a condition input reception unit configured to allow a user to input one or both of the predetermined time and the number of times of the pure water measurement operation executed by the blank measurement execution unit.

In the total organic carbon analyzer according to Clause 2, since the user inputs a time (the predetermined time) after the activation processing is started until the blank measurement is started, and/or the number of times of pure water measurement operation executed by the blank measurement execution unit by the operation of the condition input reception unit, these conditions can be set by the determination of the user.

As the predetermined time, for example, in a case where a time required for the combustion unit to reach a predetermined temperature and for the carbon dioxide amount measurement unit to be in a stable state in past measurement is known, the user sets the time as the predetermined time. As the number of times of pure water measurement operation, for example, the number of times of operations required until the measured value of the amount of carbon dioxide falls within a predetermined range in past measurement is set by the user as the number of times of pure water measurement operation to be executed this time. In addition, since the amount of CO₂ remaining attached to the combustion unit such as oxidation catalyst depends on the type of a measurement target liquid sample most recently measured, the user may change the number of operation times according to the type of the measurement target liquid sample.

### (Clause 3)

A total organic carbon analyzer according to Clause 3 further includes, in the total organic carbon analyzer according to Clause 2, a reference information display control unit configured to allow a user to input one or both of the predetermined time and the number of times of the pure water measurement operation.

In the total organic carbon analyzer according to Clause 3, by displaying the reference information, it is possible to provide a user with a determination material when a predetermined time and/or the number of times of the pure water measurement operation is set.

For the reference information for setting the predetermined time, for example, a time required for the combustion unit to reach a predetermined temperature and for the carbon dioxide amount measurement unit to be in a stable state in past measurement can be used. For the reference information for setting the number of pure water measurement operations, for example, the number of operation times required until the measured value of the amount of carbon dioxide falls within a predetermined range in past measurement, or the type of measurement target liquid sample most recently measured can be used.

### (Clause 4)

A total organic carbon analyzer according to Clause 4 is a total organic carbon analyzer such that in the total organic carbon analyzer according to any one of Clauses 1 to 3,
the liquid supply unit switches between the measurement target liquid sample, the pure water, and the inorganic acid solution to supply to the combustion unit, and
before the pure water measurement operation, the blank measurement execution unit executes inorganic acid solution using measurement of supplying the inorganic acid solution from the liquid supply unit to the combustion unit and measuring the amount of carbon dioxide by the carbon dioxide amount measurement unit.

Dilute hydrochloric acid can be suitably used for the inorganic acid solution.

With the total organic carbon analyzer according to Clause 4, when impurities adhere to an inner wall surface of a tube through which the measurement target liquid sample flows, an oxidation catalyst, or the like, dissolution and removal of the impurities can be promoted by inorganic acid solution, and the influence of the impurities at the time of measurement of the measurement target liquid sample can be suppressed. In addition, although the function of the oxidation catalyst may deteriorate after alkaline measurement target liquid sample is measured, the function of the oxidation catalyst can be recovered by passing inorganic acid solution.

### REFERENCE SIGNS LIST

- 1: Total Organic Carbon Analyzer
- 10: Liquid Supply Unit
- 11: Measurement Target Liquid Sample Reservoir
- 12: Pure Water Reservoir
- 13: Dilute Hydrochloric Acid (Inorganic Acid Solution) Reservoir
- 14: Syringe
- 141: Piston
- 15: Eight-way Valve
- 161: First Motor
- 162: Second Motor
- 20: Total Organic Carbon Measurement Unit
- 21: Combustion Unit
- 211: Combustion Tube
- 212: Oxidation Catalyst
- 213: Electric Furnace
- 22: Carrier Gas Introduction Unit
- 23: Dehumidification/Gas Treatment Unit
- 24: Carbon Dioxide Amount Measurement Unit
- 30: Control Unit
- 31: Start Operation Reception Unit
- 32: Device Activation Processing Unit
- 33: Blank Measurement Execution Unit
- 34: Sample Measurement Execution Unit
- 35: Start Operation Condition Storage Unit
- 351: Condition Input Reception Unit
- 36: Activation State Checking Unit
- 37: Blank Measurement Checking Unit
- 38: Start Operation Completion Condition Storage Unit
- 39: Reference Information Storage Unit
- 391: Reference Information Display Control Unit
- 41: Input Unit
- 42: Display Unit
- 50: Screen
- 51: Input Field
- 52: Reference Information
- 53: Condition Setting Completion Button

## Claims

1. A total organic carbon analyzer including a combustion unit configured to combust a liquid sample in a presence of a heated oxidation catalyst, a liquid supply unit configured to switch between a measurement target liquid sample and pure water to supply to the combustion unit, and a carbon dioxide amount measurement unit configured to measure an amount of carbon dioxide generated in the combustion unit, the total organic carbon analyzer further comprising:
a start operation reception unit configured to receive a predetermined start operation by a user;
a device activation processing unit configured to execute activation processing of activating the combustion unit and the carbon dioxide amount measurement unit when the start operation reception unit receives a start operation; and
a blank measurement execution unit configured to, after a predetermined time elapses from the start of the startup process or after it is detected that the combustion unit reaches a predetermined temperature and the carbon dioxide amount measurement unit is in a stable state, start a pure water measurement operation of supplying the pure water from the liquid supply unit to the combustion unit and measuring the amount of carbon dioxide in the carbon dioxide amount measurement unit, and perform control of repeatedly executing the pure water measurement operation until measured values of the amount of carbon dioxide in a latest predetermined multiple rounds fall within a predetermined range.

2. The total organic carbon analyzer according to claim 1, further comprising a condition input reception unit configured to allow a user to input one or both of the predetermined time and the number of times of the pure water measurement operation executed by the blank measurement execution unit.

3. The total organic carbon analyzer according to claim 2, further comprising a reference information display control unit configured to allow a user to input one or both of the predetermined time and the number of times of the pure water measurement operation.

4. The total organic carbon analyzer according to claim 1, wherein
the liquid supply unit switches between the measurement target liquid sample, the pure water, and the inorganic acid solution to supply to the combustion unit, and
before the pure water measurement operation, the blank measurement execution unit executes inorganic acid solution using measurement of supplying the inorganic acid solution from the liquid supply unit to the combustion unit and measuring the amount of carbon dioxide by the carbon dioxide amount measurement unit.
